# EUROPEAN PATENT SPECIFICATION

(11) **EP 3 829 564 B1**
(45) Date of publication and mention of the grant of the patent: **18.10.2023**
(21) Application number: 19768905.2
(22) Date of filing: 30.07.2019
(51) Int. Cl.: A61K 31/185, A61K 36/9066, A61K 9/20, A61P 25/00

(54) **CURCUMIN AND HOMOTAURINE FOR USE IN PREVENTING OR TREATING COGNITIVE DECLINE FORMS**
CURCUMIN UND HOMOTAURIN ZUR VERWENDUNG IN DER VORBEUGUNG ODER BEHANDLUNG VON FORMEN DES KOGNITIVEN VERFALLS
CURCUMINE ET HOMOTAURINE POUR L'UTILISATION DANS LA PRÉVENTION OU LE TRAITEMENT DE FORMES DE DÉCLIN COGNITIF

(30) Priority: 30.07.2018 IT 201800007640
(43) Date of publication of application: 09.06.2021
(73) Proprietor: ALERE VITAM S.R.L., 51034 Serravalle Pistoiese (IT)
(72) Inventor: SCAPAGNINI, Giovanni, 95131 Catania (IT); WILLCOX, Donald Graig, Urasoe City, Okinawa 901-2111 (JP); DAVINELLI, Sergio, 86100 Campobasso (IT); PREDIERI, Maurizio, 50041 Calenzano (IT)
(74) Representative: Valenza, Silvia
(86) International application number: PCT/IB2019/056476
(87) International publication number: WO 2020/026132

(56) References cited:
- WO-A2-2013/111066
- CA-A1- 2 912 611
- CN-A- 105 166 896
- US-A1- 2014 170 211

## Description

### FIELD OF THE INVENTION

The present invention refers to a composition comprising curcumin in combination with homotaurine and an edible carrier for use in prevention or treatment of cerebral functions degeneration processes or cognitive processes decline forms.

The present invention originates in the sector of nutraceutical products, food supplements.

In particular, a composition is disclosed herein for oral administration containing active ingredients suitable for preventing the slowing down or decline of cognitive processes, in particular resulting from the aging of the human organism and/or for improving the functionality of human brain.

### PRIOR ART

The increase in the average life expectancy of the population in recent decades has led to an increase in the number of people affected by some kind of cognitive disorders or slowing down of the main intellectual functions acquired.

Cognitive disorders involve a wide section of the population and generally the first signs emerge at an age above 50 years. Health statistics also show that about 5% of the population over the age of 65 and about 30% of the population over the age of 85 is affected by a cognitive disorder.

The main cognitive disorders of a mild entity include short-term memory loss, a reduction of the critical capacity and of the language.

In the cognitive disorders of a medium or high entity, instead, space-time disorientation, appreciable memory loss and a reduction in the individual's capacity to relate are found.

Generally, cognitive disorders are divided into three main types, those reversible, vascular and degenerative.

Reversible neurological disorders originate from a trauma or the interaction with an external agent, typically an infection of the meninges. These disorders usually tend to regress once the root cause is corrected.

The other two types are denoted instead as chronic pathologies and involve a progressive degeneration of the higher cerebral functions. These pathologies include cerebrovascular insufficiency, stroke, transient ischemic attack and cerebral haemorrhages and chronic brain decay, representing an irreversible involutional process that excludes the possibility of restoring the initial physiological conditions. In these cases the nutritional or pharmacological intervention is aimed at slowing down the progression of the disease and controlling the related symptoms.

In a perspective of prevention of minor brain disorders related to the aging of the organism and cognitive decay, the nutritional and dietary approach plays an important role.

This approach involves on one hand the adoption of a specific dietary regime and on the other hand the supplementation with suitable substances aimed at maintaining a correct trophism of the human brain structures.

The dietary regime and the nutritional supplementation are prepared according to the symptomatology and the age of the subject in need of treatment.

In the prevention of mild forms of cognitive decay it may be sufficient to adopt a suitable food regime or it may be convenient to supplement the diet with nutritional products.

US 2014/170211 discloses an oral composition, e.g. in form of a tablet comprising curcumin, taurine and e.g. ginko biloba extract, vitamins like vit. C, B for use in treating brain injury, mild cognitive impairment or chronic disease like Alzheimer's dementia.

The nutraceutical products currently on the market that endeavour to prevent the decay of brain and cognitive functions contain substances with antioxidant activity such as flavonoids, selenium, resveratrol, often in association with vitamins. Other products on the market contain phospholipids such as phosphatidylserine, phosphatidylcholine, phosphatidylinositol as active ingredients or omega-3 and omega-6 acids.

However, it has been observed that the use of these products provides an inadequate or non-satisfactory response.

At present, therefore, the need is felt to have available nutraceutical products or food supplements suitable for preventing the decay of cognitive functions or brain aging processes alternative to those on the market.

One of the general objects therefore consists of providing a food supplement or nutraceutical product which prevents or slows down the physiological decay of the cerebral functions.

### SUMMARY OF THE INVENTION

The present invention is defined by the claims. The inventors have found that by administering a combination of selected active ingredients a synergistic action is obtained which stimulates the cerebral structures and functions leading to a slowing down of the decay of cognitive functions in the treated subjects.

According to an embodiment, a composition is therefore provided for use in accordance to what is claimed in the appended claim 1.

Further embodiments of the composition for the use of the invention are defined in claims 2-11 appended hereto.

According to a general aspect, a composition is disclosed herein for use in the prevention and/or treatment of cognitive disorders of a mild or medium entity and the decay of the brain functions of a subject.

The subject in need of treatment may not present any symptoms of brain decay but may be interested in procrastinating the appearance of the first symptoms.

According to the present invention a composition is provided comprising a combination of curcumin and homotaurine and a physiologically acceptable vehicle and/or excipient for use in prevention or treatment of cerebral functions degeneration processes or cognitive processes decline forms. According to some embodiments, the curcumin is contained or comes from an extract of the Curcuma Longa plant, in particular from the rhizome, obtained with conventional techniques.

Typically in the composition disclosed herein the active ingredients curcumin and homotaurine are provided in an effective nutraceutical or dietary amount. According to some aspects, the composition herein disclosed finds application in the prevention of cognitive slowing down and mild forms of cognitive disorders and/or as an adjuvant in the symptoms associated with these disorders. According to some embodiments, the composition for use of the invention is a nutraceutical, a food supplement that can be introduced into the dietary regime of an individual in need of preventing or slowing down or treating the physiological decay of cognitive functions and/or the related symptoms.

The present invention will be described in detail below making reference to the following figures.

### BRIEF DESCRIPTION OF THE DRAWINGS

Some characteristics and advantages of the present invention will become clear from the appended figures in which:
Figure 1 illustrates the results of Example 3A. The bar graphs show that the combination of curcumin and homotaurine has a synergistic action in the expression of Nrf2 which remains upregulated up to 12 hours after treatment.
Figure 2 illustrates the results of Example 3B. The bar graphs highlight that the combination of curcumin and homotaurine (5, 10, 15 and 30 µM) have led to a significant (p <0.05) increase in HO-1 mRNA, measured by quantitative PCR in real time, with a maximum value at 15 µM. (Fig. 2 a, b).
Figure 3 illustrates the results of the MTT assay of Example 3c.

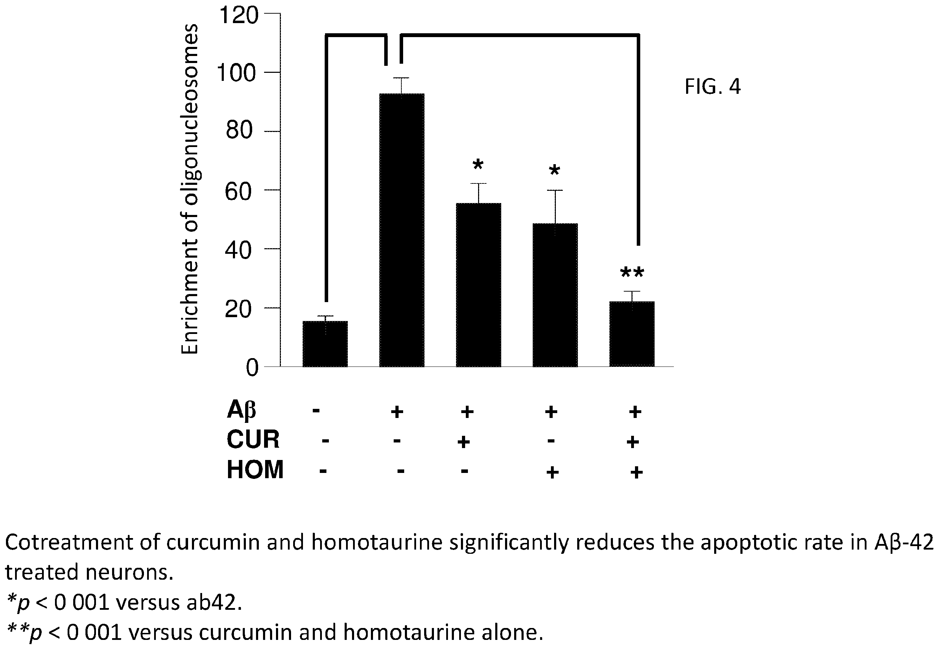

Figure 4 shows bar graphs highlighting that the combination curcumin and homotaurine reduces the apoptosis in the neurons treated with Aβ-42.
Figure 5 shows bar graphs related to the formation of toxic Aβ aggregates with curcumin (CUR), homotaurine (HOM) taken individually and in combination (CUR + HOM), as a function of the fibrillation time at 30 min (A) and 90 min (B), as described in Example 5. As can be seen from the bar graphs, the combination curcumin + homotaurine presents a synergistic activity in reducing the Aβ aggregation, with respect to the two components taken individually.

### DETAILED DESCRIPTION OF THE INVENTION

According to some aspects, the inventors have found that the combination of curcumin with homotaurine activates the transcription factor of the Nrf2 system which represents a specific biological target of cellular defence and causes the reduction of the cellular oxidative damage, in particular of neurons.

Starting from these experimental observations, the inventors have formulated a composition suitable for delaying and/or preventing cellular degeneration, in particular of the nerve cells of the brain, resulting from the physiological aging process of the organism.

It was also observed that the combined use of curcumin with homotaurine activates the cellular mitochondrial biogenesis.

By virtue of these properties and effects of the combination of the curcumin with the homotaurine, the composition herein disclosed promotes the cellular viability and the function, in particular of the nerve cells of the brain and performs an action of prevention of nerve cell deterioration resulting from degenerative processes or cellular aging.

Typically, the composition disclosed herein performs an action of prevention of the degenerative process resulting from the physiological aging of the human organism.

According to certain aspects, the inventors have found the ability of the homotaurine to significantly increase the ability of curcumin to activate the Nrf2 pathway and consequently to express repair and defence genes such as HO-1. This effect can at least partly be explained by the fact that a small sulphonic compound such as homotaurine shares with other classes of phase 2 inducers, such as electrophilic polyphenols, the ability to react with sulfhydric groups which are specific sensors for Nrf2 activation.

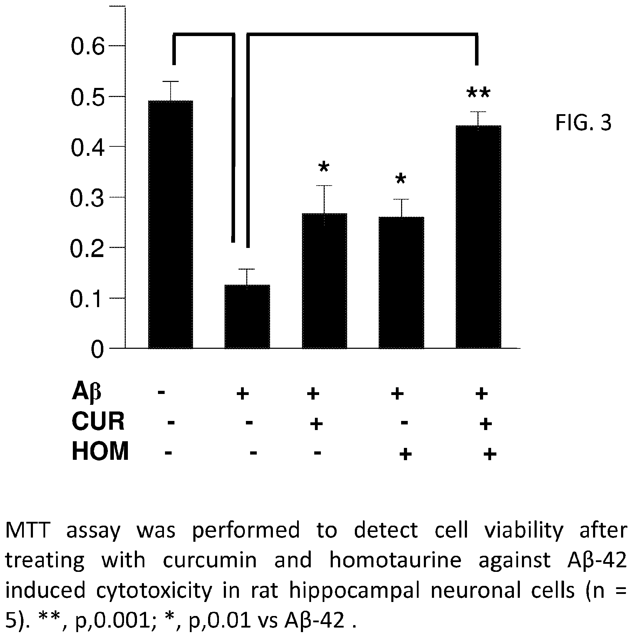

Furthermore, the inventors have determined, by means of an experimental model, as illustrated in Figure 3, the ability of curcumin and homotaurine to inhibit the amyloid beta apoptotic induction in a cell line of immortalized hippocampal neurons.

According to an embodiment of the present invention it is therefore provided a composition comprising curcumin in combination with homotaurine and an edible carrier for use in the prevention or treatment of cerebral functions degeneration processes or cognitive processes decline forms.

Curcumin represents one of the active ingredients of the composition for use of the invention.

Chemically, curcumin is a polyphenolic substance with a denomination 1,7-bis [4-Hydroxy-3-methoxyphenyl]-1,6-heptadiene-3,5-dione. Curcumin is used both in the culinary field and in the medical field in the Indian tradition.

Curcumin inhibits lipid peroxidation and intercepts and neutralizes ROS (Reactive Oxygen Species) radicals, reactive oxygen species, the most widespread free radicals such as the superoxide anion O2-, the hydrogen peroxide H2O2 and the hydroxyl radical.

Furthermore, thanks to its function of acceptor of Michael's reaction and its electrophilic characteristics, curcumin and the extracts from Curcuma Longa have demonstrated to induce the activities of Phase I and Phase II of the detox system. Furthermore, it was demonstrated that a dietary regime containing curcumin in an animal model consisting of transgenic Alzheimer's mouse (Tg2576) for 6 months, caused the suppression of the indices of the oxidative damage and the inflammation in the brain of these mice and reverses the cognitive deficits induced by Aβ amyloid. It has therefore been demonstrated that curcumin is not only an Aβ40 aggregation inhibitor better than ibuprofen and naproxen but it also prevents the formation and the toxicity of Aβ42 oligomer at a very low concentration in the range between 0.1 -11.0 microM.

Furthermore, it has been observed that curcumin passes through the blood-brain barrier and marks the plaques by inhibiting the formation of Aβ oligomers and their toxicity. The various mechanisms with which curcumin cleans amyloid, include HPS induction, functioning as a molecular chaperone that blocks the formation of protein aggregates.

According to an aspect the extract of Curcuma Longa comprises or is present in the form of complex with a soy phospholipid, such as lecithins, phosphatidylcholine, phosphatidylethanolamine, phosphatidylserine and the like, in particular soy distearoyl phosphatidylicoline. For example, these complexes, known as phytosomes, are described, for example, in EP 441 279 and are commercially available under the trade name of Fitosoma^{®}.

The lipophilic portion of this complex, the soy phospholipid, allows a better interaction of the active ingredient with the colic acids provided for the emulsion and the capture of substances to be conveyed, by absorption, to the systemic circulation. Toxicological studies carried out on animals and pharmacokinetic studies carried out on humans have indeed demonstrated the lack of toxicity and the greater absorption of the ingredient administered as a phytosome with respect to the ingredient administered in the free form.

According to some embodiments, the curcumin present in the composition disclosed herein has a titer greater than 99.5%, greater than 99.9%.

In some embodiments, the composition herein disclosed contains curcumin in an amount comprised from 50 to 5000mg, from 100 to 2000mg, from 250 to 750 mg. According to some embodiments, the composition herein disclosed contains curcumin in an amount comprised from 0.1 to 95% by weight, from 1 to 90% by weight, from 10 to 85%, from 30 to 80 by weight.

In the formulation of the composition it is possible to use curcumin of plant or synthetic origin, however it is preferred the use of curcumin lactone of plant origin, in particular obtained through extraction from a plant, in particular Curcuma Longa or portions thereof that contain it.

Curcumin can be obtained through a conventional solid-liquid extraction technique of the bioactive components from a portion of a plant that contains them, such as Curcuma Longa.

The extraction techniques that can be used are of the conventional type.

The composition for use of the invention further comprises homotaurine.

The homotaurine, 3-amino-1-propanesulfonic acid (3-APS) or tramiprosate (INN) is an aminosulfonate analogous to the taurine and GABA present in a number of species of red algae, which is also produced by way of chemical synthesis and used in the clinical field such as tramiprosate.

Homotaurine has the ability to bind to amyloidogenic proteins (β-amyloid protein), a group of proteins that organize themselves into extracellular fibrillary protein deposits from which the amyloid plaque in the brain structures originates.

Homotaurine interferes with different cellular pathways, and exerts a neuroprotective and neurotropic action through different mechanisms of action. It exerts effects against oxidative DNA damage, antifibrillogenic activity and analgesic and antinociceptive activities.

In addition to its neuroprotective effects related to the activation of the type A GABA receptors, it has been observed that homotaurine prevents the toxicity of the Aβ peptide, reducing amyloid aggregation.

Numerous clinical studies are reported in literature which were performed in order to evaluate the pharmacokinetic and pharmacodynamic properties of homotaurine, including the publication of Aisen PS, Saumier D, Briand R, Laurin J, Gervais F, Tremblay P, Garceau D. A Phase II study targeting amyloid-beta with 3APS in mild-to-moderate Alzheimer disease. Neurology. 2006 Nov 28;67(10):1757-63*.* According to some embodiments, the composition disclosed herein contains homotaurine in an amount comprised from 0.1 to 1000mg, from 5 to 500, from 20 to 100mg.

In some embodiments, the composition disclosed herein contains homotaurine, in an amount comprised from 0.01 to 40% by weight, from 0.5 to 20% by weight or from 2 to 15% by weight.

The inventors have surprisingly found the presence of a synergistic effect resulting from the combination of curcumin and homotaurine that was found by measuring biological parameters that are measurable at a cellular and organism level.

Within the context of the present invention, the term "synergism or synergistic activity" means an activity that is greater than the sum of the activities of the single active ingredient. In particular, the synergy occurs when at least two substances interact in a way in which one reinforces or increases one or more of their effects. Consequently, two substances that produce similar effects sometimes produce increased effects when used simultaneously and a quantitative evaluation is necessary for distinguishing these cases from a simple additive action (Tallarida RJ. Drug Synergism: its detection and applications. J Pharmacol. Exp. Ther. 2001 Sept.: 298(3):865-72).

The biological effects underpining the present invention are based on the modulation of cell repair and defence systems. The definition of synergism in the biological field is translated in terms of molecular events.

In some embodiments, the composition for use of the invention can comprise one or more further substances or active ingredients.

In some embodiments, the composition for use of the invention further comprises one or more vitamins, for example B group vitamins, folic acid, niacin, vitamin A, vitamin C, vitamin PP, B group vitamins being preferred.

According to some embodiments, the composition of the invention further comprises one or more trace elements and/or minerals such as salts of Mg, K, Na, Zn, Fe, Cr, Se, Mn and others, typically in the form of salts with inorganic, organic salts and in the amino chelated forms.

The composition herein disclosed may also contain extracts of black pepper, rosemary, *Vitis vinifera, Boswellia serrata, Melyssa officinalis, Ginkgo biloba.*

The composition may possibly contain essential oils such as bitter orange oil, rich in D-limonene and/or nutrients such as nucleotides from fish or yeast, resveratrol, lycopene and mixtures thereof.

The term "vehicle" as used herein indicates a medium, excipient, diluent with which the association of therapeutic or active ingredients is administered.

Any vehicle and/or excipient suitable for the desired form of preparation for administration to humans is contemplated for use with the compounds described in the present invention.

For the purposes of the present application, the term "edible" intends to indicate edible substances that are approved by health authorities for use and/or in the formulation of phytotherapeutic, nutritional or food compositions.

The term "pharmaceutically acceptable" identifies a substance that can be used in the context of pharmaceutical formulations. A physiologically acceptable vehicle may be a pharmaceutically acceptable vehicle.

Within the scope of the present application the term combination means that one or more of the active ingredients are added or mixed with one or other ingredients.

The term combination must not be understood in the sense that the active ingredients are associated with each other with formation of chemical or other types of bonds.

The compositions of the invention are suitable for food, nutritional, dietary or pharmaceutical use in mammals, in particular in humans.

The composition herein disclosed can assume a wide variety of forms of preparation, according to the desired route of administration.

The composition as herein disclosed may be in the solid form.

When the composition is presented in the solid form it may be in the form of a tablet, capsule, powder, granules.

The preparations in the solid form can comprise one or more vehicles/carriers such as for example amides, sugars, microcrystalline cellulose, and optionally diluents, granulation agents, lubricants, ligands, disintegration agents.

Typically the tablets, pills, capsules and granules may also contain a ligand such as tragacanth, acacia, corn starch or gelatin; excipients such as dicalcium phosphate; a disintegration agent such as corn starch, potato starch, alginic acid; a lubricant such as magnesium stearate; a sweetening agent such as sucrose, lactose or saccharin. If desired, the tablets can be coated using traditional techniques.

When the pharmaceutical unit form is a capsule, it can contain in addition to the materials of the aforesaid type a liquid carrier such as a fatty oil.

According to certain embodiments the composition herein disclosed contains an excipient based on cellulose that comprises i) organic esters of cellulose for example selected from cellulose acetate, cellulose propionate, cellulose triacetate, cellulose acetate propionate, cellulose acetate butyrate, ii) inorganic esters of cellulose for example selected from nitrocellulose, cellulose sulphate, iii) cellulose ethers selected from a) cellulose alkyl ethers for example selected from methyl cellulose, ethyl cellulose, ethyl methyl cellulose; b) hydroxyalkyl ethers of cellulose for example selected from hydroxyethyl cellulose, hydroxypropyl cellulose, hydroxypropyl cellulose, hydroxyethyl cellulose, hydroxypropyl methyl cellulose, ethyl hydroxyethyl cellulose; c) carboxyalkyl cellulose for example carboxy methyl cellulose, salts thereof and mixtures thereof. In certain embodiments the excipients based on cellulose are crosslinked with physiologically acceptable crosslinking agents.

In the composition there may also be flavouring agents, preservatives, colouring agents and the like.

In certain embodiments the composition for use of the is in the form of tablet. According to preferred embodiments, the composition for use of the invention is in the form of gastro-resistant tablets.

According to certain embodiments the composition of the present disclosure is in a form for the modulated or controlled release of active ingredients. By way of example, the composition may be a retard release, quick release or slow-fast release formulation. Typically, modulated or controlled release formulations contain one or more excipients based on cellulose, in particular of the type previously described.

In certain embodiments, the composition of the present disclosure comprises as an excipient a hydrogenated fatty acid, preferably having a chain with from 3 to 20 carbon atoms, from 14 to 18 carbon atoms. A typical example of a hydrogenated fatty acid is the hydrogenated palm oil.

The composition of the present disclosure may be in the liquid form.

When the composition is in the liquid form, it may be in the form of a suspension, emulsion, solution. In these cases the vehicle is liquid and can be selected for example from water, glycols, oils, alcohol and mixtures thereof.

In some embodiments, the active ingredients contained in the composition of the present disclosure may be combined or mixed as active ingredients in intimate mixture with a suitable edible vehicle and/or an excipient according to the pharmaceutical and food industry or traditional nutritional techniques.

The compositions may be suitably presented in a single pharmaceutical form and prepared using any one of the methods well known in the pharmaceutical or food technique.

The amount of active compound in the compositions of this disclosure is such that a preventive or therapeutically effective dosage will be obtained in delaying the decay process of cognitive functions.

The composition for use in the treatment and/or of prevention according to certain aspects of the invention comprise the treatment of cerebral functions degeneration processes or cognitive decline forms such as for example initial forms of senile dementia, memory loss, protection from neuronal damage for example following ischemic events.

In some embodiments, the composition disclosed herein further comprises one or more additional components such as additives, fillers, stabilizers, emulsifiers, texturizers, film-forming agents, plasticizers, wetting agents and thickeners.

Various other materials may be present as coatings or to modify the physical shape of the pharmaceutical unit. For example, the tablets can be coated with shellac, sugar or both. To prevent the breakdown during the passage through the upper part of the gastrointestinal tract, the composition may be a formulation with a gastro-resistant or enteric coating.

In certain embodiments in which the formulation disclosed herein is in the liquid form, e.g. in the cases of a syrup or elixir it may contain in addition to the association of active ingredients, saccharose as a sweetening agent, methyl and propyl-parabens as preservatives, a colouring agent and a flavouring agent as a cherry or orange flavour.

In some embodiments, in the compositions disclosed herein, the active ingredients are normally formulated in a dosage unit. The dosage unit can contain from 0.1 to 1,000 mg of each active ingredient per dosage unit for daily administration.

In some embodiments, the formulation will contain amounts of active ingredients that will depend on the severity of the cognitive decline form and the related symptoms, the condition, other therapies underway, the individual state of health and the response to the association of active ingredients.

In some embodiments the dose is in the range from 0.001% by weight to about 60% by weight of the formulation.

According to some embodiments the composition disclosed herein is a medicinal product.

According to some embodiments, the composition for use of the invention is a nutraceutical, food supplement, a nutritional product or a dietary supplement. According to another aspect, the composition disclosed herein comprises a combination of curcumin and homotaurine and a physiologically acceptable vehicle and/or excipient for use in the prevention or treatment of neurodegenerative pathologies and cognitive disorders.

According to some embodiments, the composition for use in the prevention or treatment as disclosed herein comprises the administration of an amount of an extract of Curcuma Longa titrated in curcumin from 10 to 12000 mg, typically from 20 to 1000 mg, in combination with homotaurine in an amount from 5 to 250mg, typically from 10 to 100mg per day divided into one or more, for example two or three, administrations.

Typically, the composition of the present disclosure improves brain energy metabolism and cognitive abilities after a treatment of at least 4 weeks.

Based on the studies that demonstrated the synergistic effect between curcumin and homotaurine, the applicant conducted an extensive research on cultured neuronal and endothelial cells of rat, exposing them to various concentrations (10, 25 and 50 µl / ml) of the aforesaid formulation of Example 2 for 6, 12 and 24 hours. The study demonstrated the capability of the formulation according to the invention to strongly induce the expression of heme oxygenase-1 (HO-1), a highly active protein in the defence against oxidative stress (Poon HF, Calabrese V, Scapagnini G, Butterfield DA (2004) J. Gerontol A Biol Sci Med Sci 59(5): 478-493), in the treated cells and consequently to protect the cells from the damage caused by exposure to free radicals.

Heme oxygenase is the main enzyme of heme catabolism which is degraded into biliverdin-bilirubin, carbon monoxide (CO) and iron. These products are molecules with a particularly significant biological activity: biliverdin and bilirubin have powerful antioxidant properties (Scapagnini G, D'Agata V, Calabrese V, Pascale A, Colombrita C, Alkon D, Cavallaro S. (2002) Brain Res. 954(1 ):51 -59); CO performs fundamental signalling functions and behaves like a gaseous modulator in vessel function (Abraham NG, Scapagnini G, Kappas A. (2003) J Cell Biochem. 90(6):1098-11 1 1); iron is a known gene regulator (Smith M. A., Wehr K., Harris P. L R., Siedlak S. L, Connor J. R., and Perry G. (1998) Brain Res. 788, 232-236). In addition to generating active molecules against oxidative stress, HO-1 decreases heme levels which is intrinsically a potent inductor of lipid peroxidation and free radical formation. Its activation represents an important defence mechanism for neurons exposed to oxidative damage in acute situations, such as stroke or chronic conditions, such as brain aging or during neurodegenerative diseases, such as Alzheimer's disease. In fact, both the oxidative damage and the inflammatory processes are particularly high in the brain of the patients affected by Alzheimer's disease, suggesting the potential benefit of a long-term therapy with a compound that has a powerful antioxidant and anti-inflammatory action.

The study also demonstrated that the induction of HO-1 from the formulation of the invention is related to its ability to activate Nrf2. This transcription factor is able to strongly induce the expression of other protective genes, such as type 2 detoxifying enzymes, which have high antioxidant and anti-tumor activity. Furthermore, under the same experimental conditions, the formulation according to the invention was able to considerably inhibit the apoptosis induced by beta-amyloid exposure.

A surprising aspect is however constituted by the fact that the combination of curcumin with taurine or homotaurine causes a synergistic action of the Nrf2 system which constitutes a biological target used in the experimental field as a predictive model of defence of the cell degenerative process, in particular of neurons.

Although the mechanisms leading to oxidative stress and inflammation are different, some studies indicate an important role for signalling mediated by the nuclear factor 2 transcription factor related to factor E2 (Nrf2) in the activation of cellular protection. Nrf2, a member of the family of the Cap'n'Collar transcription factors, is sequestered in the cytoplasm by binding to the Kelch ECH protein which associates protein 1 (Keap1) under unstimulated conditions. Keap1 plays a central role in the regulation of the Nrf2 response. Under normal conditions, Nrf2 is a Keap1 target, which promotes Nrf2 degradation in the proteasome through interactions with a ubiquitin ligase. Keap1 also acts as a sensor of stress signals, through stress-induced oxidation of key cysteine residues that lead to conformational changes and the inability to bind Nrf2. Numerous stimuli, including oxidative stress and electrophilic compounds, such as polyphenols, lead to the destruction of the Nrf2/Keap1 complex, freeing Nrf2 for translocation in the nucleus where it interacts with basic transcription factors of the leucine hinge such as the members of the families Maf and Jun and binds to a cis-agent element, the antioxidant response element (ARE, also called EpRE or OSRE). Nrf2-ARE is an important metabolic pathway that regulates phase II antioxidant reactions, triggering the simultaneous expression of numerous enzymes and scavengers. The ARE is found in several inducible cytoprotective genes linked to the so-called cellular stress response, such as glutathione-S-transferase, heme oxygenase 1 (HO-1), glutamate cysteine ligase, ferritin, gamma-glutamyl cysteine synthetase, NAD (P) H quinone oxidoreductase thioredoxin and peroxiredoxins, and this confirms Nrf2 as a central element in the cellular survival response. With the constant activation of this complex genetic machinery, it is therefore possible to gradually repair all the damage as soon as they arise. Based on these tests, the modulation of Nrf2 and HO-1 in different contexts, including brain, skin and vessels, has been recently suggested as a potential pharmaceutical strategy for the treatment and prevention of degenerative disorders and pathological aging. The model was used by the applicant to determine the possibility that curcumin and homotaurine are synergistic in preventing ROS-induced damage (reactive oxygen species) or, more generally, cell death related to damage, since each of them activates a metabolic pathway of the cell in response to the lesion. More specifically, they have a specific ability to synergistically activate the nuclear transcription factor Nrf2. Nrf2 interacts with the ARE sequence at the promoter level of several inducible genes with critical defensive and reparative properties, with consequent cellular overexpression of such genes, an improvement in cellular defences and an inhibition of cell death. Many electrophilic polyphenols have demonstrated to activate the Nrf2/ARE metabolic pathway, including curcumin (Scapagnini et al, Mol Neurobiol 2011).

The following examples are mainly provided to illustrate the present invention and do not intend to limit the scope of protection which is defined by the appended claims.

### EXAMPLE 1

Composition in the form of tablets for the prevention and treatment of cognitive slowing down having the following formulation:

| Active ingredients: | |
|---|---|
| Extract of Curcuma Longa | 500 mg |
| Homotaurine | 50 mg |

| Excipients: | |
|---|---|
| Hydroxymethylpropyl cellulose | 2.0 g |
| Carnauba wax | 0.1 g |

### EXAMPLE 2

Composition in capsule for the treatment having the following formulation
a) Extract of Curcuma Longa: from 50 to 2000 mg;
b) Homotaurine: from 25 to 100 mg.

### EXAMPLE 3

To evaluate the neuroprotective and anti-aging activity of the combination of homotaurine and curcumin taken individually and their synergistic combination as disclosed herein, the following experiments have been carried out.

### 3A) Activation of Nrf2 expression in different cells.

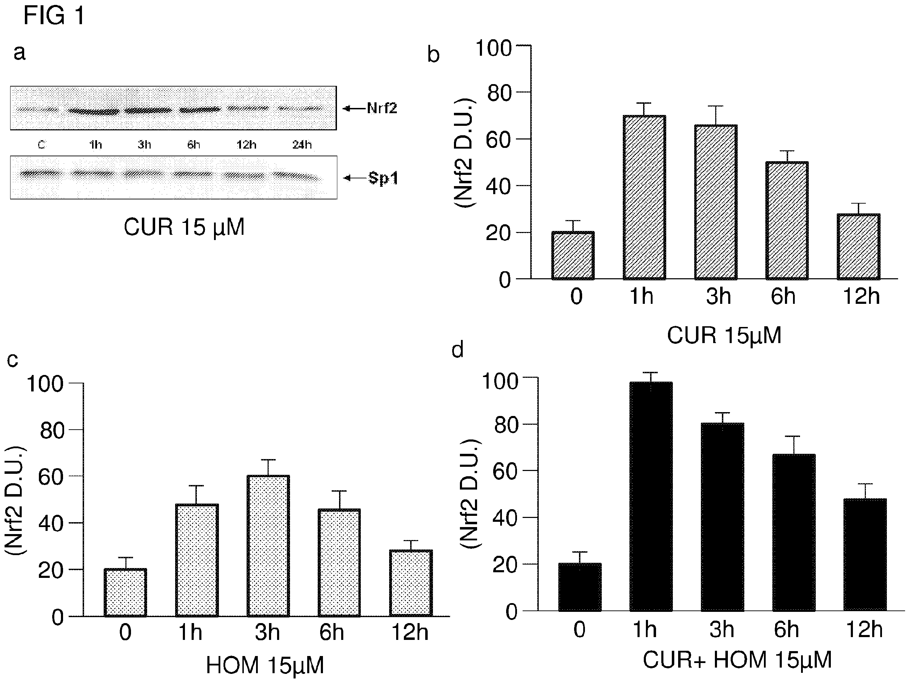

Three different cell lines, cortical neurons, astrocytes and endothelial cells, were exposed to curcumin, homotaurine or a combination of the two compounds at the final concentration of 25 µM to evaluate the expression of the Nrf2 protein over time. As shown in Figure 1, the treatment with curcumin caused a significant timedependent increase in the expression of the Nrf2 protein in nuclear extracts. The quantification of three independent Western blots showed that after 1 hr of exposure to 25 µM of curcumin, the Nrf2 expression increased significantly and remained upregulated for up to 12 hours, while the levels of the constitutive gene of the transcription factor Sp1 were stable. The homotaurine induced a lower increase in nuclear Nrf2. The combination of curcumin and homotaurine significantly induced Nrf2 expression.

### 3B) Upregulation of HO-1 and phase II detoxification enzymes in different cells.

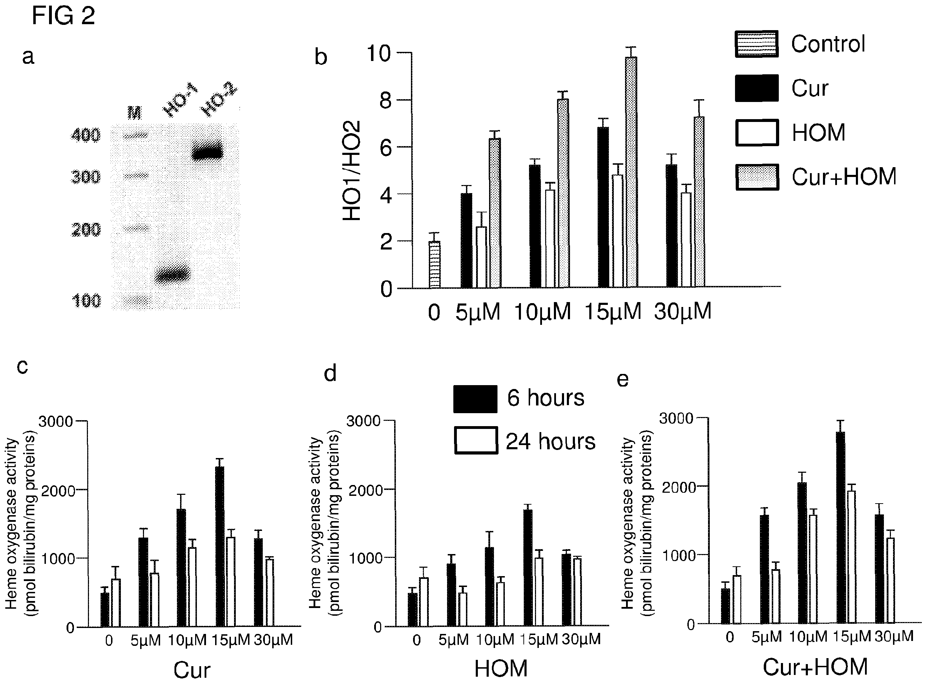

The exposure of three different cultured cell lines, cortical neurons, astrocytes and endothelial cells, for 6 hours at different concentrations of curcumin, homotaurine or a combination of the two compounds (5, 10, 15 and 30 µM) led to a significant (p <0.05) increase in HO-1 mRNA, measured by quantitative PCR in real time, with a maximum value at 15 µM. (Fig. 2 a, b). The curcumin induces a dosedependent increase in HO-1 mRNA, measured relative to the non-inducible paralogue HO-2 gene, which reaches the maximum (about 6 times) at 15 µM and subsequently decreases to 30 µM. The homotaurine, on the other hand, is much less active in inducing the expression of the HO-1 gene at the same concentrations and again reaches the maximum at 15 µM. To confirm that the expression of the HO-1 gene measured at the mRNA level corresponded to an equivalent increase in heme oxygenase activity, this activity was measured at the same doses of curcumin and homotaurine 6 and 24 hours after administration. The pattern of increase in the activity was comparable to the results obtained by looking at cellular mRNAs, confirming the functional significance of the data obtained from PCR determinations in real time (Fig. 2 c, d, e).

### 3C) Neuroprotective effects of the invention

The experimental field chosen, to test the ability of compounds to improve cell survival, was a model of cell death induced in rat hippocampal neurons exposed to amyloid beta 42.

Aβ 42 at a 20 µM concentration is able to induce evident cytotoxicity in neuronal cells as indicated by the MMT assay. The respective concentrations of curcumin and homotaurine were optimally selected at 15 µM for the subsequent evaluation of their potential synergistic effect against neuronal damage induced by Aβ 42. An ELISA kit for detecting the cell death was used to determine oligonucleosomal fragments of the cytoplasmic DNA associated with cell death by apoptosis. The enrichment of oligonucleosomes released into the cytoplasm was calculated as the absorbance of the sample cells /absorbance of the control cells. The administration of curcumin plus homotaurine demonstrated a synergistic effect in terms of apoptosis reduction. The results represent the ± ESM mean of three independent experiments. ANOVA was performed followed by Tukey's post hoc test to determine the significance level.

### EXAMPLE 4

### Cell culture

The rat hippocampal neurons (H 19-7) were purchased by the American Type Culture Collection (Manassas, VA, USA) and cultured according to the manufacturer's instructions.

### Preparation of the nuclear extract and Western blot for Nrf2

The neurons were washed twice with 1X PBS. The cells were then collected in 1 ml of 1X PBS and centrifuged at 3000 rpm for 3 min at 4°C. The cell pellet was carefully resuspended in 200 ml of cold buffer containing HEPES 10 mM (pH 7.9), KCI 10 mM, EDTA 0.1 mM, EGTA 0.1 mM, DTT 1 µM and complete protease inhibitor cocktail (Roche, Mannheim, Germany).

The pellet was then incubated on ice for 15 minutes to allow the cells to swell. After this time, 15 µl of 10% of NP-40 were added and the tube was vortexed for 10 s. The homogenate was then centrifuged at 3000 rpm for 3 min at 4°C and the nuclear pellet was resuspended in 30 µl of cold buffer consisting of HEPES 20 mM (pH 7.9), NaCl 0,4 M, EDTA 1 mM, EGTA 1 mM, DTT 1 µM and protease inhibitors. The pellet was then incubated on ice for 15 minutes and vortexed for 10-15 s every 2 minutes. The nuclear extract was finally centrifuged at 13,000 rpm for 5 minutes at 4°C. The supernatant containing the nuclear proteins was resolved with SDS-polyacrylamide gel and subjected to immunoblot analysis using anti-Nrf2 antibodies (dilution 1: 500) and anti-Sp1 (dilution 1: 500).

### MTT assay

The MTT assay was used to measure the viability of neuronal and retinal cells.

Briefly, the cells were plated in a 96-well plate in DMEM-F12 with 7% of FBS. After the culture in DMEM-F12 without serum for 12 hours, Aβ 42 (20 µM) alone or in combination with homotaurine (HOM), Curcumin (Cur) or HOM + Cur were added to the medium. Twenty-four hours later, the cells were incubated with 10 mL of MTT (Roche, 0.5 mg/ml) at 37u for 4 hours, and then the crystals were dissolved with 150 mL of DMSO. The absorbance at 570 nm was detected by a spectrophotometer (TECAN Infinite M200, Switzerland).

### EXAMPLE 5

### Preamble

The cerebral deposition of amyloid β-peptide (Aβ) in the brain is an invariant feature of Alzheimer's disease (AD). Aβ is a main constituent of senile plaques in AD. The neurotoxicity derives from the conformational transition of Aβ from random-coil to beta-sheet and from its oligomerization.

### Comparative experimental example

In the present study, the fluorescence spectroscopy with thioflavin T was used to examine the effects of curcumin (CUR), homotaurine (HOM) taken individually and a combination of the two compounds as disclosed herein on the formation of toxic Aβ aggregates.

Thioflavin-T (Th-T dosage), analysis for the inhibition of the Aβ 42 aggregation (also referred to AB42).

Beta-amyloid (Aβ 1-42) aggregation studies were conducted using the SensoLyte ThT Ab42 aggregation kit (AnaSpec, Inc, Fremont, CA).

The Aβ42 peptide was dissolved in a cold analysis buffer (4 °C) and allowed to hydrate for a few minutes. The solution was centrifuged at 10,000 rpm for 5 minutes at 4°C to centrifuge any precipitated material. The fibrillation reaction was initiated by adding thioflavin dye (ThT) at a concentration of 2 mmol/L.

Three test samples (CUR; HOM; CUR + HOM) were added in a volume of 5 µL and the reaction was started by adding the Aβ42 peptide solution. The final volume of the test samples was 100 µL. At the same time, the positive control and the negative control containing nordihydroguaiaretic acid (NDGA), a well-defined inhibitor of Ab aggregation, at the final concentration of 100 µmol/L were established. Fluorescence intensity was measured at 37°C with Ex/Em ¼ 440 nm/484 nm and by shaking of 15s between readings using the microplate reader (Synergy H1; BioTek, Winooski, VT). The fluorescence intensity measurements expressed as relative fluorescence units (RFU) were taken for 90 minutes at 5-minute intervals.

### Results

Effects of CUR, HOM and CUR + HOM on Aβ aggregation depending on the fibrillation time (A) 30 min, (B) 90 min. NDGA at a concentration of 100 µmol/L was used as an inhibitor of the Aβ aggregation (p <.001). The results are presented as a ± DS mean of three or four fluorescence intensity measurements (Ex/Em ¼ 440 nm / 484 nm every 5 minutes at 37°C).

Both CUR and HOM at the concentrations tested, significantly influence the fibrillation reaction for the entire measurement time with respect to the control. Surprisingly, the combination of the two compounds (1/1 ratio) at the final concentration of 100 and 200 µmol/L demonstrated a synergistic activity in reducing Aβ aggregation.

* p <.001 vs. control, ** p <.001 vs. CUR and HOM alone.

The two-way ANOVA analysis showed that at 30 and 90 minutes of fibrillation reaction CUR + HOM 15 and 30 µmol/L significantly inhibited the reaction with respect to CUR and HOM only at the same concentration.

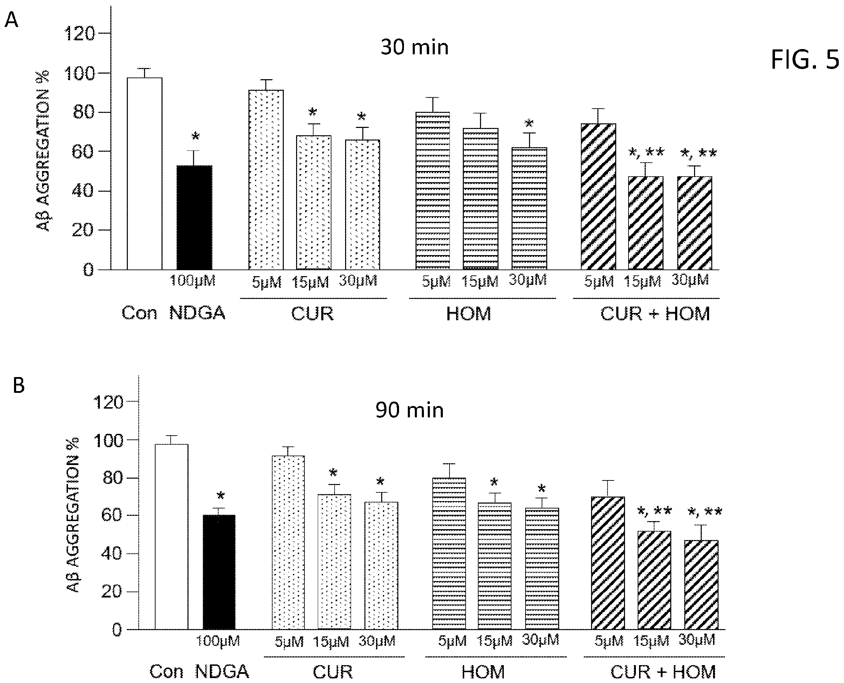

These results are shown in the appended Figure 5 A, B.

## Claims

1. A composition comprising curcumin in combination with homotaurine and an edible carrier for use in prevention or treatment of cerebral functions degeneration processes or cognitive processes decline forms.

2. The composition for use according to claim 1, wherein the cognitive processes decline forms are initial forms of senile dementia or memory loss.

3. The composition for use according to any one of claims 1-2, wherein curcumin is obtained from an extract of *Curcuma Longa.*

4. The composition for use according to any one of claims 1-3, comprising one or further substances selected from B group vitamins, folic acid, niacin, vitamin A, vitamin C, vitamin PP, trace elements selected from salts of Mg, K, Na, Zn, Fe, Cr, Se, Mn, plant extracts selected from extracts of black pepper, rosemary, *Vitis vinifera, Boswellia serrata, Melyssa officinalis, Ginkgo biloba* and mixtures thereof.

5. The composition for use according to any one of claims 1-4, wherein curcumin is in the form of complex with a soy phospholipid.

6. The composition for use according to claim 5, wherein the soy phospholipid is selected from lecithins, phosphatidylcholine, phosphatidylethanolamine, phosphatidylserine, in particular soy distearoyl phosphatidylicoline.

7. The composition for use according to any one of claims 1 to 6, **characterized in that** it is in the form for oral administration.

8. The composition for use according to claim 7 **characterized in that** it is in the form of a capsule or tablet, preferably gastro-resistant.

9. The composition for use according to any one of claims 1 to 8, **characterized in that** it is a dietary supplement, a nutraceutical or a food supplement.

10. The composition for use according to anyone of claims 1-9 wherein the cerebral functions degeneration is cerebrovascular insufficiency, stroke, transient ischemic attack, cerebral haemorrhages or chronic brain decay.

11. The composition for use according to claim 1 in form of a capsule having the following formulation
a) Extract of Curcuma Longa: from 50 to 2000 mg;
b) Homotaurine: from 25 to 100 mg.

## Patentansprüche

1. Zusammensetzung, umfassend Curcumin in Kombination mit Homotaurin und einem essbaren Träger zur Verwendung in der Vorbeugung oder Behandlung von Degenerationsprozessen der Gehirnfunktionen oder Formen von Verfall der kognitiven Prozesse.

2. Zusammensetzung zur Verwendung nach Anspruch 1, wobei es sich bei den Formen von Verfall der kognitiven Prozesse um anfängliche Formen von seniler Demenz oder Gedächtnisverlust handelt.

3. Zusammensetzung zur Verwendung nach einem der Ansprüche 1-2, wobei Curcumin aus einem Extrakt von *Curcuma Longa* erhalten wird.

4. Zusammensetzung zur Verwendung nach einem der Ansprüche 1-3, umfassend eine oder weitere Substanzen, ausgewählt aus Vitaminen der B-Gruppe, Folsäure, Niacin, Vitamin A, Vitamin C, Vitamin PP, Spurenelementen, ausgewählt aus Salzen von Mg, K, Na, Zn, Fe, Cr, Se, Mn, Pflanzenextrakten, ausgewählt aus Extrakten von schwarzem Pfeffer, Rosmarin, *Vitis vinifera, Boswellia serrata, Melyssa officinalis, Ginkgo biloba* und Mischungen davon.

5. Zusammensetzung zur Verwendung nach einem der Ansprüche 1-4, wobei Curcumin in Form eines Komplexes mit einem Soja-Phospholipid vorliegt.

6. Zusammensetzung zur Verwendung nach Anspruch 5, wobei das Soja-Phospholipid aus Lecithinen, Phosphatidylcholin, Phosphatidylethanolamin, Phosphatidylserin, insbesondere Sojadistearoylphosphatidylicolin ausgewählt ist.

7. Zusammensetzung zur Verwendung nach einem der Ansprüche 1 bis 6, **dadurch gekennzeichnet, dass** sie in der Form zur oralen Verabreichung vorliegt.

8. Zusammensetzung zur Verwendung nach Anspruch 7, **dadurch gekennzeichnet, dass** sie in Form einer Kapsel oder Tablette, vorzugsweise magensaftresistent, vorliegt.

9. Zusammensetzung zur Verwendung nach einem der Ansprüche 1 bis 8, **dadurch gekennzeichnet, dass** es sich um ein Nahrungsergänzungsmittel, ein Nutrazeutikum oder ein Lebensmittelergänzungsmittel handelt.

10. Zusammensetzung zur Verwendung nach einem der Ansprüche 1-9, wobei es sich bei der Degeneration der Gehirnfunktionen um zerebrovaskuläre Insuffizienz, Schlaganfall, transitorische ischämische Attacke, zerebrale Blutungen oder chronischen Hirnzerfall handelt.

11. Zusammensetzung zur Verwendung nach Anspruch 1 in Form einer Kapsel mit der folgenden Formulierung
a) Extrakt von Curcuma longa: von 50 bis 2000 mg;
b) Homotaurin: von 25 bis 100 mg.

## Revendications

1. Une composition comprenant de la curcumine en combinaison avec de l'homotaurine et un support comestible pour une utilisation dans la prévention ou le traitement de processus de dégénérescence des fonctions cérébrales ou de formes de déclin des processus cognitifs.

2. La composition pour une utilisation selon la revendication 1, dans laquelle les formes de déclin des processus cognitifs sont des formes initiales de démence sénile ou de perte de mémoire.

3. La composition pour une utilisation selon l'une quelconque des revendications 1-2, dans laquelle la curcumine est obtenue à partir d'un extrait de *Curcuma Longa.*

4. La composition pour une utilisation selon l'une quelconque des revendications 1-3, comprenant une ou plusieurs substances choisies parmi les vitamines du groupe B, l'acide folique, la niacine, la vitamine A, la vitamine C, la vitamine PP, les oligo-éléments choisis parmi les sels de Mg, K, Na, Zn, Fe, Cr, Se, Mn, des extraits de plantes choisis parmi les extraits de poivre noir, romarin, *Vitis vinifera, Boswellia serrata, Melyssa officinalis, Ginkgo biloba* et leurs mélanges.

5. La composition pour une utilisation selon l'une quelconque des revendications 1-4, dans laquelle la curcumine se présente sous la forme d'un complexe avec un phospholipide de soja.

6. La composition pour une utilisation selon la revendication 5, dans laquelle le phospholipide de soja est choisi parmi les lécithines, la phosphatidylcholine, la phosphatidyléthanolamine, la phosphatidylsérine, en particulier la distéaroyl phosphatidylicoline de soja.

7. La composition pour une utilisation selon l'une quelconque des revendications 1-6, **caractérisée par le fait qu'**elle se présente sous une forme pour l'administration orale.

8. La composition pour une utilisation selon la revendication 7 **caractérisée par le fait qu'**elle se présente sous la forme d'une capsule ou d'un comprimé, de préférence gastro-résistant.

9. La composition pour une utilisation selon l'une quelconque des revendications 1-8, **caractérisée par le fait qu'**il s'agit d'un supplément diététique, d'un produit nutraceutique ou d'un complément alimentaire.

10. La composition pour une utilisation selon l'une quelconque des revendications 1-9, dans laquelle la dégénérescence des fonctions cérébrales est une insuffisance vasculaire cérébrale, un accident vasculaire cérébral, un accident ischémique transitoire, des hémorragies cérébrales ou une détérioration chronique du cerveau.

11. La composition pour une utilisation selon la revendication 1 sous la forme d'une capsule ayant la formulation suivante
a) Extrait de Curcuma Longa : de 50 à 2000 mg ;
b) Homotaurine : de 25 à 100 mg.
